# EUROPÄISCHE PATENTSCHRIFT

(11) **EP 2 953 592 B1**
(45) Veröffentlichungstag und Bekanntmachung des Hinweises auf die Patenterteilung: **19.07.2017**
(21) Anmeldenummer: 14749356.3
(22) Anmeldetag: 03.02.2014
(51) Int. Cl.: A61F 5/14, B29D 35/12, B29D 35/14, A43D 1/02, A43B 7/14, A43B 17/14

(54) **VERFAHREN ZUR HERSTELLUNG VON INDIVIDUELL ANGEPASSTEN SCHUHEINLAGEN**
METHOD FOR PRODUCING CUSTOMIZED INSOLES
PROCEDE DE FABRICATION DE SEMELLES ORTHOPEDIQUES INDIVIDUALISEES

(30) Priorität: 06.02.2013 DE 102013002012
(43) Veröffentlichungstag der Anmeldung: 16.12.2015
(73) Patentinhaber: Stumpf, Jürgen, 36037 Fulda (DE)
(72) Erfinder: Stumpf, Jürgen, 36037 Fulda (DE)
(74) Vertreter: Hebing, Norbert
(86) Internationale Anmeldenummer: PCT/DE2014/000038
(87) Internationale Veröffentlichungsnummer: WO 2014/121780

(56) Entgegenhaltungen:
- DE-A1- 3 835 008
- DE-A1- 4 404 695
- US-A- 5 687 467
- US-A1- 2005 285 302

## Beschreibung

Die Erfindung bezieht sich auf ein Verfahren zur Herstellung von individuell angepassten Schuheinlagen.

Derartige Schuheinlagen werden für vielfältige Zwecke benötigt. Ein Hauptanwendungsgebiet liegt im Bereich der Orthopädie. Durch individuell angepasste Schuheinlagen können Haltungsschäden und dergl. ausgeglichen werden. Schuheinlagen werden auch für die Diabetesversorgung benötigt. An Diabetes erkrankte Personen benötigen eine weiche Fußbettung, damit Verletzungen der Fußsohle möglichst vermieden werden. Ein weiterer Bereich ist die Anpassung von Sportschuhen an die individuellen Bedürfnisse eines Sportlers.

Derartige Einlagen werden häufig manuell gefertigt, wobei ein Spezialist Lagen aus unterschiedlichem Material, wie Polyurethan, Kork und Filz, sowie Stützelemente und Pelotten zusammenklebt. Dabei ist ein wiederholtes Anprobieren notwendig, um eine möglichst genaue Anpassung an den Fuß der jeweiligen Person zu erreichen.

Um den Aufwand hierfür zu verringern, sind auch schon konfektionierte Schuheinlagen aus einem thermoplastischen Polyurethanschaum, die im Spritzgussverfahren hergestellt werden, bekannt. Aber auch diese müssen noch an den Fuß des Trägers angepasst werden. Ein Beispiel für die Vorgehensweise beim Anpassen ist in der DE 10 2010 015 145 A1 beschrieben. Die Sohle wird erwärmt und in einen Schuh eingelegt. Beim Gehen mit diesem Schuh erfolgt eine Anpassung an die Fußsohlenkontur des Trägers. Dieses Verfahren kann aber nicht ganz überzeugen, da bei einer zu geringen Aufwärmung des Materials keine Verformung erfolgt und bei einer zu starken Erwärmung das Polyurethan schmilzt und seine elastische Funktion verliert.

In der DE 38 35 008 A1 wird ein Verfahren beschrieben, bei dem zunächst die Druckverteilung einer Fußsohle mittels einer flexiblen Messmatte erfasst wird. Aus den gewonnenen Werten wird aus einem Rohling ein den Messwerten entsprechendes individuelles Fußbett gefräst.

Gemäß der DE 4 404 695 A1 sowie gemäß der US 5,687,467 wird jeweils ebenfalls ein Fräsverfahren zur Herstellung einer orthopädischen Einlage verwendet.

Die US 2005/0285 302 A1 beschreibt die Herstellung einer Gussform, die durch mehrfache Verwendung zu Herstellung einer Vielzahl von Schuhsohlen genutzt werden kann.

Die Erfindung beruht somit auf der Aufgabe, einen Prozess darzustellen, bei dem auf kostengünstig Weise individuell angepasste aus Polyurethan bestehende Schuheinlagen geschaffen werden, die keiner Nachbearbeitung mehr bedürfen. Zur Lösung des Problems sieht die Erfindung vor, dass ein Verfahren zur Herstellung von individuell angepasste aus Polyurethan bestehenden Schuheinlagen die folgenden Schritte aufweist:
Erstellung eines Fuß-Datensatzes, der wenigstens die Geometrie der Oberfläche einer Fußsohle beschreibt,
Berechnen eines Maschinen-Datensatzes für eine Fräsmaschine aus dem Fuß-Datensatz,
Fräsen des Nests und des Deckels einer Spritzgussform gemäß dem Maschinen-Datensatz aus jeweils einem Block aus einem nicht metallischen Material, das einschmelzbar und wieder verwertbar ist,
Einbringung der Spritzgussform in eine Spritzgussmaschine und
Ausspritzen der Spritzgussform mit einem Polyurethan zur Herstellung einer einzigen geschäumten Schuheinlage,
Entnahme der Schuheinlage aus der Spritzgussform und Überführung der Spritzgussform in einen Schmelzofen,
Einschmelzen ein oder mehrerer Spritzgussformen und Herstellung von neuen Blöcken in einem Gussverfahren.

Das Verfahren basiert darauf, dass eine individuell zu dem Fuß einer bestimmten Person passende Spritzgussform geschaffen wird, die lediglich die einmalige Herstellung einer einzigen exakt passenden Schuheinlage verwendet. Eine solche Vorgehensweise, bei der die Spritzgussform nach einer einmaligen Verwendung gleich wieder zerstört wird, ist aber nur dann möglich, wenn die Herstellung der Spritzgussform kostengünstig ist. Bei üblichen Formen, die aus einem dauerhaltbaren Material, z. B. aus Aluminium, hergestellt werden und die für die Massenproduktion einer immergleichen Einlage verwendet werden, sind die Herstellungskosten zu hoch.

Die Schuheinlage besteht vorzugsweise aus einem Duroplast z. B. einem Polyurethan. Die Verwendung eines Duroplasts, das sich nicht thermisch verformt, gewährleistet eine größere und beständige Formstabilität.

Die Erfindung sieht daher weiterhin vor, dass das Nest und der Deckel einer Spritzgussform aus jeweils einem Block aus einem nichtmetallischen Material, das einschmelzbar und somit wieder verwertbar ist, geschaffen werden. Das Material wird somit so gewählt, dass es einerseits stabil genug ist, zumindest dem Druck, der beim einmaligen Ausspritzen der Spritzgussform entsteht, standzuhalten. Es soll aber weiterhin so weich sein, dass es möglichst mit einem geringen Kraftaufwand gefräst werden kann und die Fräswerkzeuge eine möglichst lange Standzeit haben. Des Weiteren soll der Energieaufwand zum Einschmelzen gering sein und das Material soll beim Einschmelzen nicht degenerieren, so dass dasselbe Material möglichst lange für immer neue Spritzgussformen genutzt werden kann.

Spritzgussformen, die aus diesem Material hergestellt werden, werden erfindungsgemäß nur einmal genutzt und werden danach eingeschmolzen, um aus dem eingeschmolzenen Material neue Blöcke zu schaffen.

Vorzugsweise wird bei der Berechnung des Maschinendatensatzes nicht nur der Fußdatensatz herangezogen, sondern gleichzeitig auch ein Schuhdatensatz, der die inneren Abmessungen des Schuhs beschreibt, in der die Einlage Verwendung finden soll.

Die Schuheinlage erhält somit eine an die Fußsohle des Trägers angepasste Form und gleichzeitig eine Außenrandkontur, die zum Schuh passt, so dass sie lagegenau im Schuh fixiert ist.

Applikationen wie Verstärkungseinlagen, Dämpfungseinlagen, pelottenförmige Erhebungen und dergl. werden vor dem Aufspritzen in die Spritzgussform eingebracht, so dass diese mit dem eingespritzten Material eine innige Verbindung eingehen und eine einheitliche Einlage entsteht.

Wie weiter oben beschrieben wurde, muss das nichtmetallische Material, aus dem die Spritzgussformen hergestellt werden, bestimmte Eigenschaften erfüllen. Ein wachsartiges Material hat sich hierfür bewährt. Dieses kann durch Zugabe von Bindemitteln und dergl. ausreichend stabilisiert werden, damit die gewünschten Eigenschaften erreicht werden.

Eine weitere Maßnahme erleichtert die Handhabung der Blöcke deutlich. Diese verfügen über Längsnuten an ihrer Außenseite.

Diese Längsnuten passen zu entsprechenden Schlitzen in der Spritzgussmaschine, so dass die Blöcke in die Spritzgussmaschine über eine Schiebeverbindung eingesetzt und gehalten werden. Die Längsnuten werden vorzugsweise in der Gussform erzeugt. Dadurch können die Blöcke auch schon in der Fräsmaschine über eine Schiebeverbindung gehalten werden. Des Weiteren braucht in der Fräsmaschine lediglich eine Seite des Blocks bearbeitet werden, nämlich die Ausformung der Nestkontur und der Deckelkontur.

Im Folgenden soll anhand eines Ausführungsbeispiels die Erfindung nochmals näher erläutert werden. Dazu zeigt die
- Fig. 1: ein Ablaufdiagramm und die
- Fig. 2: den Querschnitt durch einen Block.

Der erste Schritt 1 im Prozess besteht aus dem Erfassen der Fußdaten der Person, deren Schuhe mit einer Einlage zur Fußbettung versehen werden.

Dazu wird die Fußsohle mit einem zweidimensionalen Scanner abgetastet, so dass ein zweidimensionales Bild der Fußsohle entsteht.

Des Weiteren wird ein dreidimensionaler Abdruck gebildet. Dazu drückt die Person ihren Fuß in einen Schaumblock, der damit ein exaktes dreidimensionales Bild der Fußsohle wiedergibt. Dieser kann ebenfalls gescannt werden, so dass die Geometrie der Fußsohle in einer Vielzahl von Koordinatenpunkten vorliegt, die den Fußdaten-Satz bilden.

Ergänzend kann eine Druckverteilung in der Fußsohle bestimmt werden. Zusätzlich kann der Fuß als Ganzes mit Hilfe eines Videoaufnahmesystems gescannt werden, so dass geometrische Daten des gesamtes Fußes und nicht nur der Fußsohle vorliegen.

In einem weiteren Schritt 2 wird aus diesen Daten die Form einer Schuheinlage errechnet und daraus die notwendigen geometrischen Formen einer Spritzgussform. Bei der Berechnung dieser Daten können die Daten über den Schuh, in dem die Einlage verwendet wird, einfließen, so dass insbesondere die Außenrandkontur der Einlage bestimmt wird. Aus der Form der Schuheinlage wird ein Maschinen-Datensatz für eine Fräsmaschine errechnet.

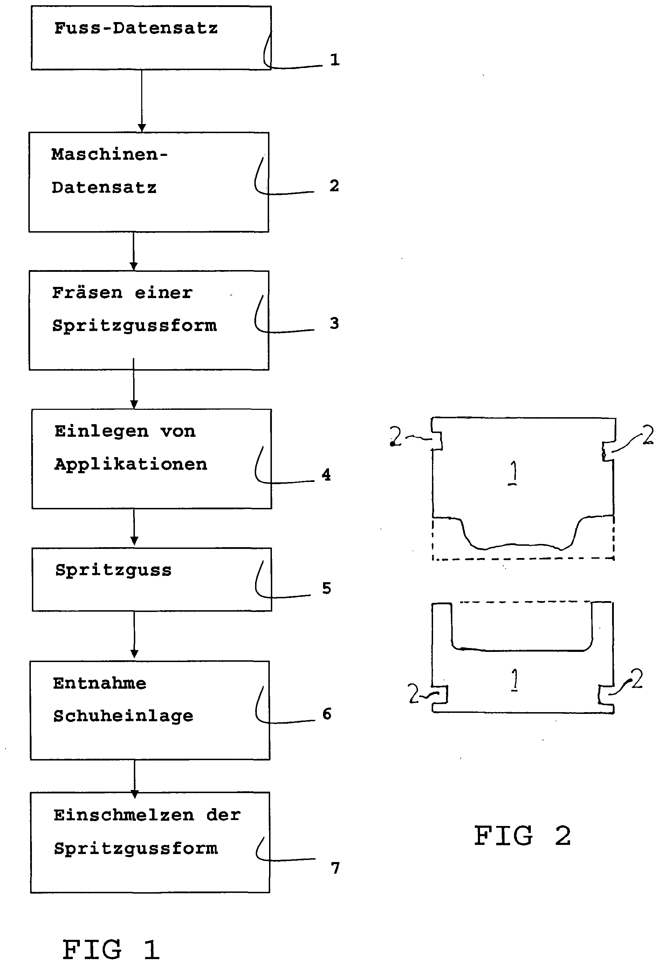

In einem weiteren Schritt 3 werden zwei Blöcke, die über Längsnuten verfügen, und wie sie in der Fig. 2 dargestellt sind, zur Verfügung gestellt und in eine Fräsmaschine eingestellt. Mit Hilfe des Maschinen-Datensatzes wird aus dem einen Block ein Nest und aus dem anderen Block ein Deckel einer Spritzgussform gefräst.

Nest und Deckel werden in einem nächsten Schritt 4 in eine Spritzgussmaschine eingebracht, wobei dort gegebenenfalls Verstärkungseinlagen oder Pelotten in die Spritzgussform eingebracht werden, die von dem Polyurethan umschäumt werden sollen. Im nächsten Schritt 5 wird die Form mit einem Polyurethan ausgespritzt. Die fertige Spritzform wird nach dem Erkalten entnommen (Schritt 6). Von der Form müssen lediglich Spritzgussränder abgetrennt werden. Ansonsten entspricht diese Spritzform - ohne weitere Nachbearbeitung - exakt der Fußform, die der Träger benötigt.

Die beim Spritzguss verwendeten Blöcke werden nach dieser einmaligen Verwendung im nächsten Schritt 7 gesammelt und einem Schmelzofen zugeführt. Dort werden sie eingeschmolzen, um anschließend in einem Gussverfahren zu neuen Blöcken geformt zu werden.

Wie der Fig. 2 zu entnehmen ist, besitzen die Blöcke 1 Längsnuten 2, die beim Gießen erzeugt werden. Mit diesen Längsnuten 2 werden sie sowohl in der Fräsmaschine als auch in den Spritzgussmaschinen in entsprechende Führungen eingeführt, so dass sowohl das Einbringen in die Fräsmaschine als auch in die Spritzgussmaschinen relativ rasch erfolgen kann. Weitere Festigungsmittel wie Schrauben oder dergl. werden nicht benötigt.

## Patentansprüche

1. Verfahren zur Herstellung von individuell angepassten Schuheinlagen, aufweisend die folgenden Schritte:
Erstellung eines Fuß-Datensatzes, der wenigstens die Geometrie der Oberfläche einer Fußsohle beschreibt,
Berechnen eines Maschinen-Datensatzes für eine Fräsmaschine aus dem Fuß-Datensatz,
Fräsen des Nests und des Deckels einer Spritzgussform gemäß dem Maschinen-Datensatz aus jeweils einem Block aus einem nicht metallischen Material, das einschmelzbar und wieder verwertbar ist,
Einbringung der Spritzgussform in eine Spritzgussmaschine und
Ausspritzen der Spritzgussform mit einem Polyurethan zur Herstellung einer einzigen geschäumten Schuheinlage,
Entnahme der Schuheinlage aus der Spritzgussform und Überführung der Spritzgussform in einen Schmelzofen,
Einschmelzen einer oder mehrerer Spritzgussformen und Herstellung von neuen Blöcken in einem Gussverfahren.

2. Verfahren nach Anspruch 1, **dadurch gekennzeichnet, dass** die Schuheinlage aus einem Duroplast besteht.

3. Verfahren nach Anspruch 1 oder 2, **dadurch gekennzeichnet, dass** die Berechnung des Maschinen-Datensatzes für eine Fräsmaschine aus einem Fuß-Datensatz und einem Schuh-Datensatz erfolgt.

4. Verfahren nach einem der vorhergehenden Ansprüche, **dadurch gekennzeichnet, dass** Applikationen vor dem Ausspritzen in die Spritzgussform eingebracht werden.

5. Verfahren nach einem der vorhergehenden Ansprüche, **dadurch gekennzeichnet, dass** das nicht metallische Material, das für die Herstellung der Blöcke verwendet wird, ein wachsartiges Material ist.

6. Verfahren nach einem der vorhergehenden Ansprüche, **dadurch gekennzeichnet, dass** jeder Block an der Außenseite Längsnuten aufweist.

7. Verfahren nach Anspruch 6, **dadurch gekennzeichnet, dass** die Längsnuten in der Gussform erzeugt sind.

## Claims

1. Method for the production of customized insoles, comprising the following steps:
Creating a foot dataset that at least describes the geometry of the surface of the sole of a foot,
calculating from the foot dataset a machine dataset for a milling machine,
milling of a mould cavity and a lid of an injection mould on the basis of the machine dataset from at least one block of non-metallic material that is meltable and reusable,
introducing the injection mould into an injection moulding machine and
hosing of the injection mould with a polyurethane for producing a single foamed insole,
extracting the insole from the injection mould and transferring the injection mould into a melting furnace,
melting one or more injection moulds and manufacturing new blocks in a casting process.

2. Method according to claim 1, **characterized in that** the insole is made of a thermosetting plastic material.

3. Method according to claim 1 or 2, **characterized in that** the machine data set for a milling machine is calculated from a foot data set and a shoe data set.

4. Method according to one of the preceding claims, **characterized in that** appliqués are introduced into the injection mould before the mould is hosed.

5. Method according to one of the preceding claims, **characterized in that** the non-metallic material that is used for manufacturing the blocks is a wax-like material.

6. Method according to one of the preceding claims, **characterized in that** each block has longitudinal grooves on the outside.

7. Method according to claim 6, **characterized in that** the longitudinal grooves are created in the casting mould.

## Revendications

1. Procédé pour la fabrication de semelles orthopédiques adaptées individuellement, présentant les étapes suivantes:
la création d'un jeu de données d'un pied, lequel décrit au moins la géométrie de la surface d'une plante du pied,
le calcul d'un jeu de données machine pour une fraiseuse à partir du jeu de données d'un pied,
le fraisage de la cavité et du couvercle d'un moule d'injection conformément au jeu de données machine respectivement à partir d'un bloc en un matériau non métallique pouvant être fondu et recyclé,
l'insertion du moule d'injection dans une machine de moulage par injection, et
l'injection du moule d'injection avec un polyuréthane pour la fabrication d'une unique semelle orthopédique expansée,
le prélèvement de la semelle orthopédique du moule d'injection et le transport du moule d'injection dans un four de fusion,
la fonte d'un ou plusieurs moules d'injection et la fabrication de nouveaux blocs dans un procédé de moulage.

2. Procédé selon la revendication 1, **caractérisé en ce que** la semelle orthopédique se compose d'une matière thermodurcissable.

3. Procédé selon la revendication 1 ou 2, **caractérisé en ce que** le calcul du jeu de données machine pour une fraiseuse s'effectue partir d'un jeu de données d'un pied et d'un jeu de données d'une chaussure.

4. Procédé selon l'une des revendications précédentes, **caractérisé en ce que** des applications sont insérées dans le moule d'injection avant l'injection.

5. Procédé selon l'une des revendications précédentes, **caractérisé en ce que** le matériau non métallique, lequel est utilisé pour la fabrication des blocs, est un matériau semblable à de la cire.

6. Procédé selon l'une des revendications précédentes, **caractérisé en ce que** chaque bloc présente des rainures longitudinales sur le côté extérieur.

7. Procédé selon la revendication 6, **caractérisé en ce que** les rainures longitudinales sont produites dans le moule.
